(19) Europäisches Patentamt
European Patent Office
Office européen des brevets

(11) Publication number : 0 468 866 A1

(12) # EUROPEAN PATENT APPLICATION

(21) Application number : 91402022.7

(22) Date of filing : 19.07.91

(51) Int. Cl.5 : C07F 9/6561, A61K 31/675, C07F 9/6512

(30) Priority : 24.07.90 EP 90402129

(43) Date of publication of application : 29.01.92 Bulletin 92/05

(84) Designated Contracting States : AT BE CH DE DK ES FR GB GR IT LI LU NL SE

(71) Applicant : MERRELL DOW PHARMACEUTICALS INC.
2110 East Galbraith Road
Cincinnati Ohio 45215-6300 (US)

(72) Inventor : Halazy, Serge
2 Rue Hans Haug
Wolfisheim, F-67200 Strasbourg (FR)

(74) Representative : Gillard, Marie-Louise et al
Cabinet Beau de Loménie 55, Rue d'Amsterdam
F-75008 Paris (FR)

(54) **Novel carbocyclic analogs of certain nucleosides.**

(57) This invention relates to novel carbocyclic analogs of certain nucleosides, to the process for their preparation and to their use as anti-viral agents.

EP 0 468 866 A1

This invention relates to novel carbocyclic analogs of certain nucleosides, to the process for their preparation and to their use as anti-viral agents.

AIDS is an immunosuppressive or immunodestructive disease that predisposes subjects to fatal opportunistic infections. Characteristically, AIDS is associated with a progressive depletion of T-cells, especially the helper-inducer subset bearing the $OKT_4$ surface marker.

Human immunodeficiency virus (HIV) has been reproducibly isolated from patients with AIDS or with the symptoms that frequently precede AIDS. HIV is cytopathic and appears to preferentially infect and destroy T-cells bearing the $OKT_4$ marker, and it is now generally recognized that HIV is the etiological agent of AIDS.

Since the discovery that HIV is the etiological agent of AIDS, numerous proposals have been made for anti-HIV chemotherapeutic agents that may be effective in treating AIDS sufferers. Thus, for example, European Patent Specification No. 196 1 85 describes 3′-azido-3′-deoxythymidine (which has the approved name zidovudine), its pharmaceutically acceptable derivatives and their use in the treatment of human retrovirus infections including AIDS and associated clinical conditions. Vince et al., Antiviral Research, 9 -(1/2), 120 (1988) describes certain carbocyclic purine nucleosides (in particular(±)-9-(cis-4-(hydroxymethyl)-2-cyclopentenyl) guanine and their use against HIV.

World wide, hepatitis B virus (HBV) is a viral pathogen of major consequence. It is most common in Asian countries, and prevalent in sub-Saharan Africa. The virus is aetiologically associated with primary hepatocellular carcinoma and is thought to cause 80% of the world's liver cancer. In the United States more than ten thousand people are hospitalized for HBV illness each year, an average of 250 die with fulminant disease.

The United States currently contains an estimated pool of 500,000 - 1 million infectious carriers. Chronic active hepatitis will develop in over 25% of carriers, and often progresses to cirrhosis. It is estimated that 5,000 people die from HBV-related cirrhosis each year in the USA, and that perhaps 1,000 die from HBV-related liver cancer. Even when a universal HBV vaccine is in place, the need for effective anti-HBV compounds will continue. The large reservoir of persistently infected carriers, estimated at 220 million worldwide, will receive no benefit from vaccination and will continue at high risk for HBV induced liver disease. This carrier population serves as the source of infection of susceptible individuals perpetuating the instance of disease particularly in endemic areas or high risk groups such as intravenous drug abusers and homosexuals. Thus, there is a great need for effective antiviral agents, both to control the chronic infection and reduce progression to hepatocellular carcinoma.

Clinical effects of infection with the HBV virus range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease as outlined above. In "Viral infections of Humans" (second edition, Ed., Evans, A.S. (1982) Plenum Publishing Corporation, New York), Chapter 12 describes in some detail, the aetiology of hepatitis infections.

Hepatitis B virus (HBV) is a small DNA containing virus which infects humans. It is a member of the class of closely related viruses known as the hepadnaviruses, each member of which selectively infects either mammalian or avian hosts, such as the woodchuck and the duck. Recent insights into the mechanism of replication of the hepadnavirus genome indicate the importance of reverse transcription of an RNA intermediate, suggesting that the reverse transcriptase is a logical chemotherapeutic target. Other logical targets include herpes viruses, e.g. herpes simplex virus I and II (HSV-I and HSV-II), cytomegalovirus (CMV) and varicella-zoster-virus (VZV), as well as respiratory syncitial viruses are also specific targets for end-use application of the compounds of this invention.

It is an object of this invention to use the below defined carbocyclic analogs of certain purine and pyrimidine nucleosides as defined by Formula I, for the treatment or prophylaxis of viral infections such as for example hepatitis B, retroviral infections, especially AIDS, respiratory syncitial viruses, and herpes viruses such as herpes simplex I and II, cytomegalovirus and varicella-zoster-virus.

More specifically this invention relates to compounds of the formula

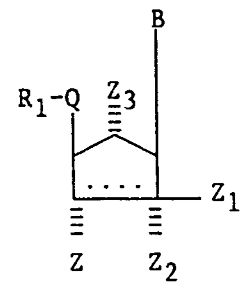

I

the tautomers, optical and geometric isomers and mixtures thereof, and the pharmaceutically acceptable salts thereof, wherein

B is a purine or a pyrimidine moiety of the sub-formulae

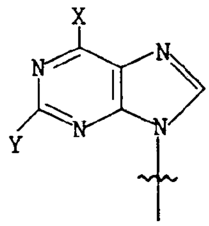

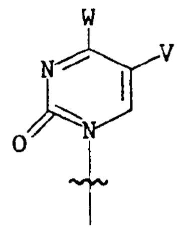

the depicted wavy line illustrating the point of attachment of the B moiety to the remaining portion of Formula I, and the dotted line represents a facultative double bond,

X is $-OR_2$, $-N(R_2)(R_2)$, Cl, -SH or $-SCH_3$, with $R_2$ being H, $C_{1-6}$ alkyl, or $C_{3-6}$ cycloalkyl,

Y is H or $NH_2$,

W is $NH_2$, OH or $-OC_{1-3}$ alkyl,

V is H, $C_{1-3}$ alkyl, halogeno, $-N(R_2)(R_2)$, -SH, -SCH3, -C≡CH, $-CH=CH_2$, or CH=CHBr,

Z is H, F or $N_3$, with the proviso that when the dotted line represents a double bond then Z is H, and each of $Z_1$, $Z_2$ and $Z_3$ is H, F, or OH, with the proviso that when one of $Z_1$ or $Z_2$ is OH the other is H, and with the further proviso that when the dotted line is a double bond, $Z_1$ is H or F and $Z_2$ is deleted,

$R_1$-Q is $-CH_2OCH_2P(O)(OR)_2$, $-CH_2C(A)(A)P(O)(OR)_2$, or $-CH=C(A)P(O)(OR)_2$, and A is H, F or Cl, and R is H or $C_{1-6}$ alkyl.

The term $C_{1-6}$ alkyl includes the straight and branched chain hydrocarbyl radicals including methyl, ethyl, propyl, isopronyl, butyl, t-butyl, pentyl and hexyl. The term halogeno embraces F, Cl, Br and I, and the term $-N(R_2)(R_2)$ embraces amino, mono- and di-$C_{1-6}$ alkyl amines. The term $R_1$-Q, as expressed in Formula I is defined as $-CH=C(A)P(O)(OR)_2$, $-CH_2OCH_2P(O)(OR)_2$ or-$CH_2C(A)(A)P(O)(OR)_2$ in order to avoid any ambiguity as to the bonding of the Q moiety to the $R_1$ and cyclopentyl moieties. However, throughout this specification, for the convenience of better understanding, the $R_1$ and Q moieties, when the context dictates, will be used and defined separately,

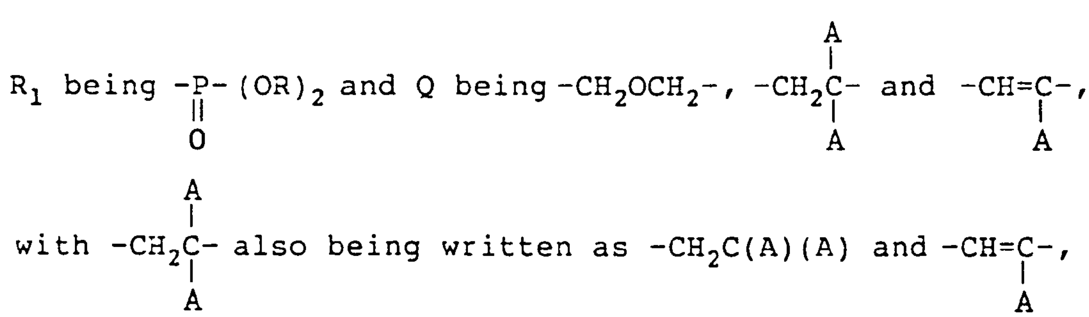

$R_1$ being ... and Q being $-CH_2OCH_2-$, ... and ...,

with ... also being written as $-CH_2C(A)(A)$ and ...,

being also written as -CH=C(A)-.

For convenience in describing the preparation of the compounds of Formula I, the moiety -CH=C(A) will

also be referred to as $Q_1$, $-CH_2C(A)(A)$ will be referred to as $Q_2$, and $-CH_2OCH_2$ will also be referred to as $Q_3$, and $Q_4$ will be used to embrace both -CH2C(A)(A) and $-CH_2OCH_2$. The $R_1$ moiety may also be written as $P(O)(OR')_2$, with R′ representing a $C_{1-6}$ alkyl, preferably ethyl or isopropyl.

The expression "pharmaceutically acceptable acid addition salts" is intended to apply to any non-toxic organic or inorganic acid addition salts of the base compounds of formula 1. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. Either the mono- or the di-acid salts can be formed, and such salts can exist in either a hydrated or a substantially anhydrous form. The acid salts are prepared by standard techniques such as by dissolving the free base in an aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic forms, demonstrate higher melting points and an increased stability.

The optical and geometrical isomers of the compounds of formula I may best be prepared by processes designed to enrich the production of the desired geometrical isomers and/or the optical isomers. However, mixtures may be resolved or isolated according to conventional and standard procedures well known in the art, e.g. chromatographic separation, fractional crystallization, use of optically active acids, enzymatic resolution and the like. Tautomeric enol-keto forms may exist at the 6-position of the purine nucleus, and the pyrimidine will exhibit amide-imine tautomeric forms.

The preparation of the compounds of this invention may be effected by standard chemical processes and techniques analogously known and well understood by those of ordinary skill in the art using carbocyclic nucleosides as starting materials which are known and described in the above-cited reference edited by Evans. Other references useful in the preparation of this invention are Shealy Y.F., O'Dell, C.A., Shannon W.M., Arnett G., J. Med. Chem. 1984, 27, 1416; A. Borthwick and coworkers, J. Med. Chem. 1991, 34, 907; A. Borthwick and coworkers, J. Med. Chem. 1990, 33, 179; Marquez V.E., Liu M.I., Med. Res. Rev. 6, 1 (1986). In general the route synthesis for any individual compound will depend on the specific substituents of each molecule and upon the ready availability of intermediates necessary; again such factors being appreciated by those of ordinary skill in the art.

It is to be noted that the compounds of this invention include both X- and Y-substituted purines and W- and V-substituted pyrimidines. For convenience in illustrating the preparation of the compounds embraced by Formula I, only the X,Y-substituted purines will be depicted. However, in all instances it is to be understood that the analogous chemistry is applicable to the W,V-substituted pyrimidines. Thus, in those instances wherein a structural formula depicts either a X,Y-substituted purine or a X′,Y′-substituted purine, then by analogy the same type of reaction(s) is applicable to a W,V-substituted pyrimidine or a W′,V′-substituted pyrimidine wherein the W′ and V′ moieties (as well as the X′ and Y′ moieties) are those which are either left intact (i.e., they would not be altered by the reaction conditions illustrated and described for the preparation of any sub-group or specific compounds) or else they are precursors which are readily transformable to the desired W and V substituents (or the desired X and Y substituents) once the desired molecules have been synthesized and all that is necessary is to transform the precursor substituent to the desired substituent and to de-esterify the protected phosphonate moiety; the sequence of these last 2 steps being optional under most conditions.

In general, all of the compounds of Formula I may be prepared using the following general reaction scheme.

REACTION SCHEME A

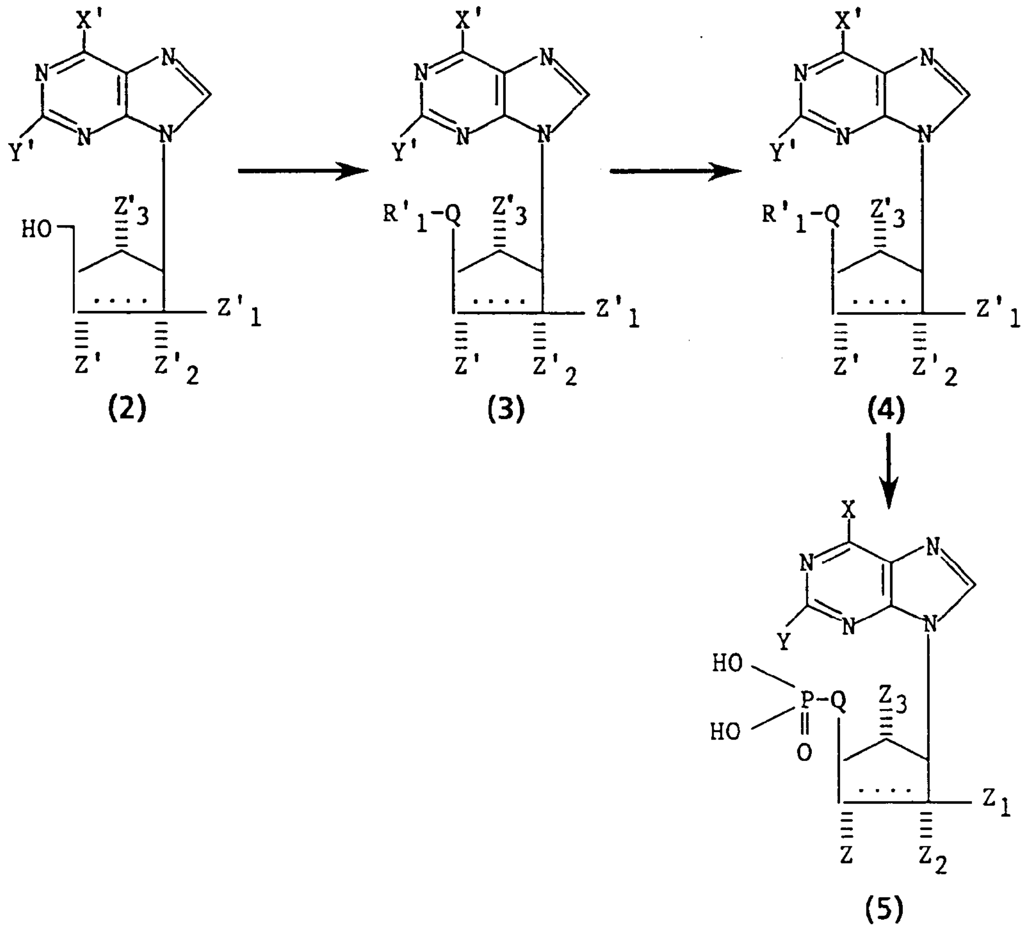

wherein

X', Y', Z', $Z'_1$, $Z'_2$, $Z'_3$ are precursors which, when necessary, are transformable to the previously defined X, Y, Z, $Z_1$, $Z_2$ and $Z_3$ moieties respectively, or are the desired X, Y, Z, Z, $Z_2$ and $Z_3$ moieties of any given compound.

$R'_1$ is $P(O)(OC_{1-6}$ alkyl$)_2$, and

Q is as previously defined.

Exemplary of the type of transformation of the precursors (2) is the case wherein it is desired to produce a compound wherein at least one of Z, $Z_1$, $Z_2$ or $Z_3$ is OH, the 5'OH moiety would first be selectively protected by treatment with one equivalent of t-butyldimethylsilyl chloride to protect the 5'OH group and then any other OH group of the Z moieties may be protected by such protecting groups as, for example, acetyl, benzyl, benzoyl or tetrahydropyran. Once the OH groups of Z, $Z_1$, $Z_2$ or $Z_3$ are protected, the 5'OH t-butyl dimethyl silyl moiety is selectively removed by reaction with tetrabutyl ammonium fluoride ($Bu_4NF$) in tetrahydrofuran to give the 5'OH which is then ready for the appropriate reaction to couple the desired $R'_1Q$ moiety to the 4' position of the cyclopentyl or cyclopentenyl moiety. Following this, the removal of any OH protecting group of the Z moieties, the transformation of the X' and Y' moieties to the desired X and Y moieties, as defined by Formula I, and the de-esterification of the $R'_1$ protecting groups of the phosphonate group, such as by reaction with trimethylsilylbromide (TMSBr) (or its iodide) in anhydrous $CH_3CN$, DMF or $CH_2Cl_2$, followed by treatment with water, may be effected.

The art also teaches that there are numerous starting materials which may be utilized in initiating the preparation of the purine and pyrimidine derivatives defined in Formula I and, therefore, the X',Y', and W',V'precursor moieties transformable to the desired X,Y and W,V substituents (or left intact as the case may be) is meant to include all such possibilities. For example, if X is to be chloro in the final product, then X' would include chloro for that could be left intact throughout the entire series of reactions for any given compound. On the other hand, one could utilize a 6-chloro or a 2,6-dichloro purine starting compound and after the series of reactions is completed they could be transformed into a 6-amino or a 2,6-diamino final product, respectively by reaction with

the appropriate amines [i.e.,$NH_3$, $-HN(H)(R_2)$ or $-HN(R_2)(R_2)$] in methanol under pressure at about 100°C. Similarly, in those instances wherein X is OR, the corresponding 6-chloro purine moiety may be reacted with an acid (HCl/HOH) to obtain the corresponding 6-OH analog or with an alcohol in basic conditions to obtain the corresponding 6-OR analog. In the same vein, X′ may be a protected OH group using such groups as $-OCH_3$, $OCH_2CH_2OCH_3$, Obenzyl, O-C(O)phenyl, or -O-C(O)methyl and then such moieties would be transformed to the desired 6-OH moieties. Analogously, a compound wherein X is to be an amine, the precursor transformable thereto could be an amide (e.g.,NHC(O)phenyl), an amidine (e.g.

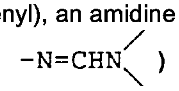 )

or $-N(C(O)phenyl)_2$, said moieties being transformable to the desired amine by standard procedures. Similarly, a 6-chloro purine starting material may be transformed to the desired $-SCH_3$ moiety by treatment with methyl mercaptan using standard procedures. These reactions, and the proper sequencing of such reactions, all follow principles well understood and practiced by those skilled in the art and all steps used processes well known in the art, as well as other factors such as the ready availability of the appropriate starting materials, reactants and the volume capacity of compound to be produced are also factors which are appreciated by those skilled in the art for choosing any particular reaction scheme.

The foregoing generic reaction scheme may be further illustrated by the following reaction schemes directed to the various Q moieties.

In the instance wherein it is desired to prepare compounds of the sub-formula

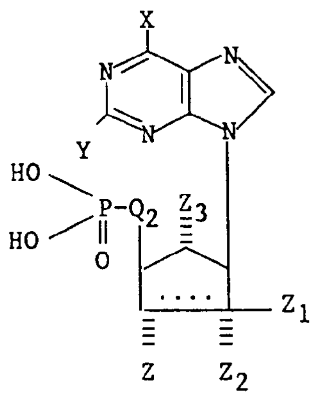

(6)

wherein X, Y, Z, $Z_1$, $Z_2$, $Z_3$ and $Q_2$, and the 2′,3′-dotted line of the cyclopentyl moiety is a facultative double bond are as previously defined, the following reaction scheme would be applicable.

REACTION SCHEME B

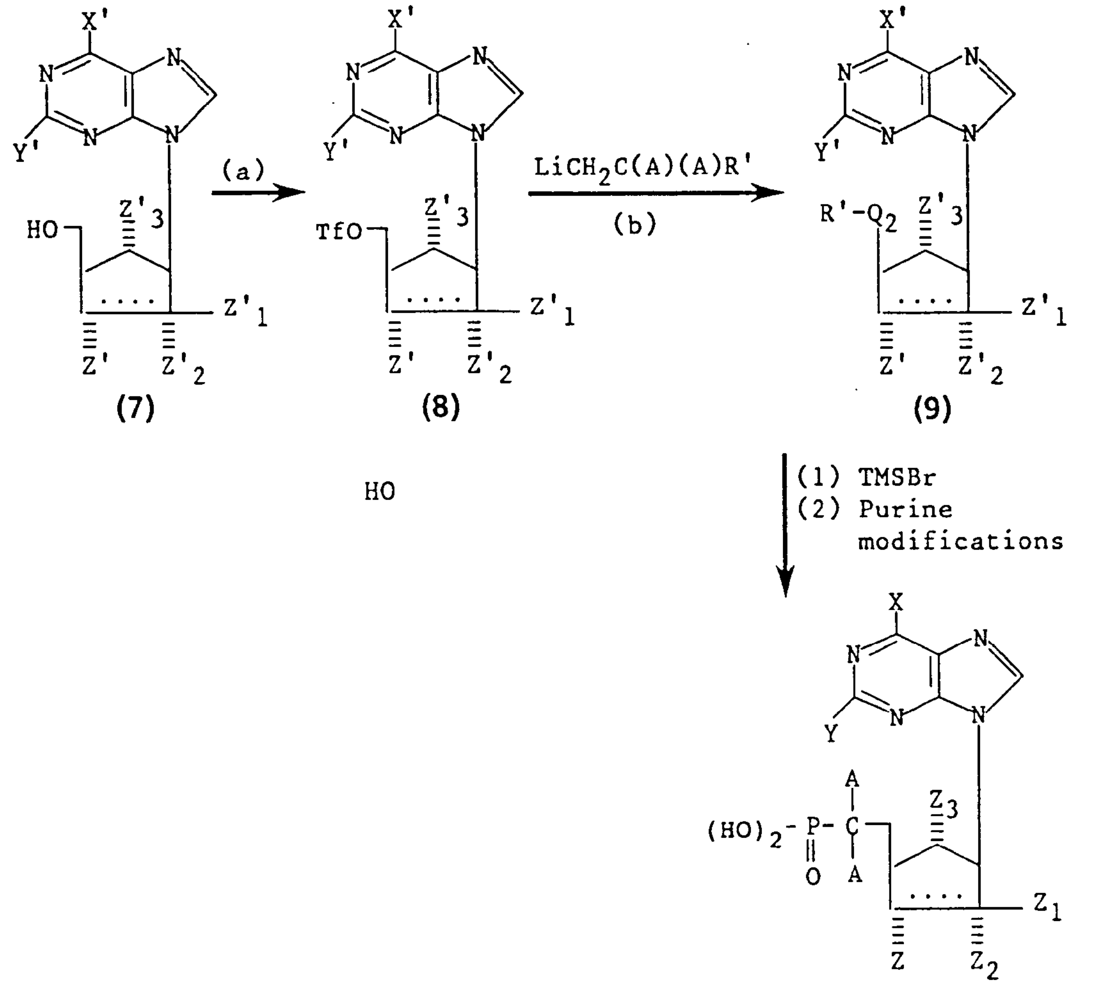


(7) (8) (9) (6)

wherein X', Y', A, Z, $Z_1$, $Z_2$ and the dotted line are as previously defined.

In Step (a) of this reaction the 5'OH moiety is converted to its triflate by treatment with triflic anhydride ($Tf_2O$), (i.e., $F_3CS(O)_2OS(O)_2CF_3$) in pyridine, using $CH_2Cl_2$ as a solvent and the resulting triflate is reacted with a lithio derivative of the diethyl ester of the appropriately A,A-di-substituted phosphonic acid derivative (e.g., $LiCF_2P(O)(OEt)_2$) to produce compounds of Formula (9). Products of Formula (9) are de-esterified with TMSBr and the X',Y' precursor substituents, if necessary, of the purine base are modified to produce the desired compounds with the desired X,Y substituents. Of course, if desired, the de-esterification may take place after the modifications to the purine base are effected.

To prepare compounds of the formula

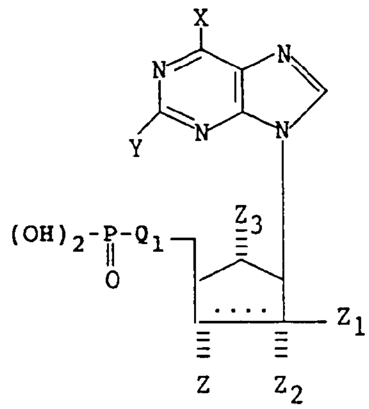

(10)

wherein the X, Y, Z, $Z_1$, $Z_2$, $Z_3$ and $Q_1$ substituents are as previously defined, the following reaction sequence may be employed

REACTION SCHEME C

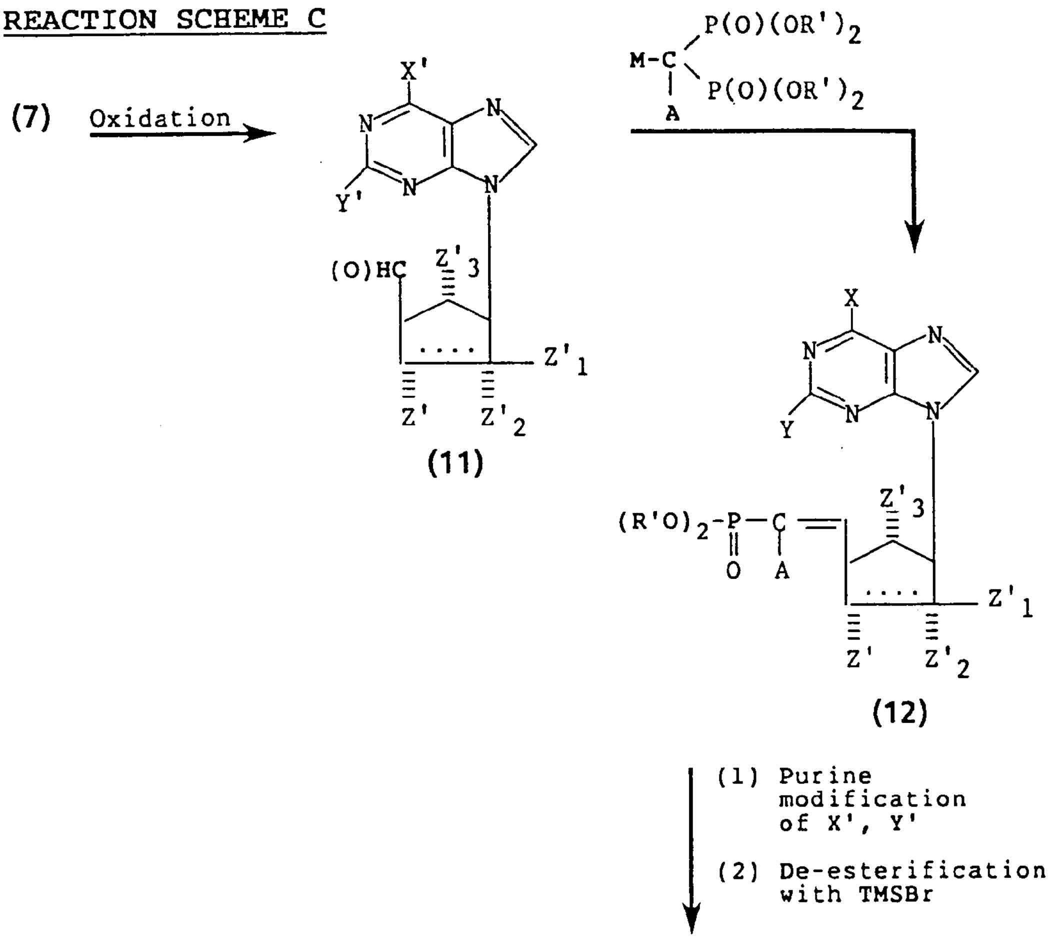


(10)

wherein M is an alkali metal, preferably Na or Li, and X', Y', X, Y, A, R', Z, $Z_1$, $Z_2$, and $Z_3$ are as previously defined.

The foregoing reaction involves the oxidation of the 5'-OH function to its corresponding aldehyde, using pyridinium chlorochromate in an anhydrous solvent (e.g., dichloromethane) under an inert atmosphere (argon) at about 20°C. The resulting product (11) is reacted with an alkali metal salt, preferably sodium of the A-substituted methyl di-phosphonate (e.g., sodium salt of tetraethylmethylene diphosphonate) in anhydrous tetrahydrofuran at about 20°C, and followed by quenching with aqueous ammonium chloride to produce compounds (12) which are then subjected to the usual modification of the X′,Y′-substituents of the purine base and the de-esterification of the phosphonate ester with TMSBr.

To prepare compounds of the formula

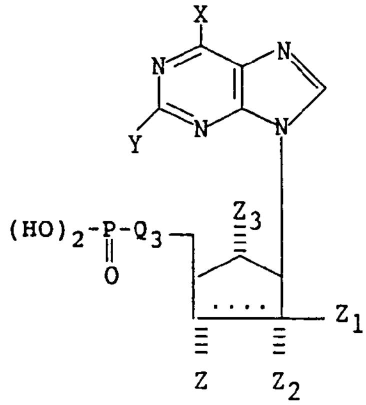

(13)

wherein X, Y, Z, $Z_1$, $Z_2$, $Z_3$ and $Q_3$ are as previously defined, the following series of reactions may be utilized in their preparation.

REACTION SCHEME D

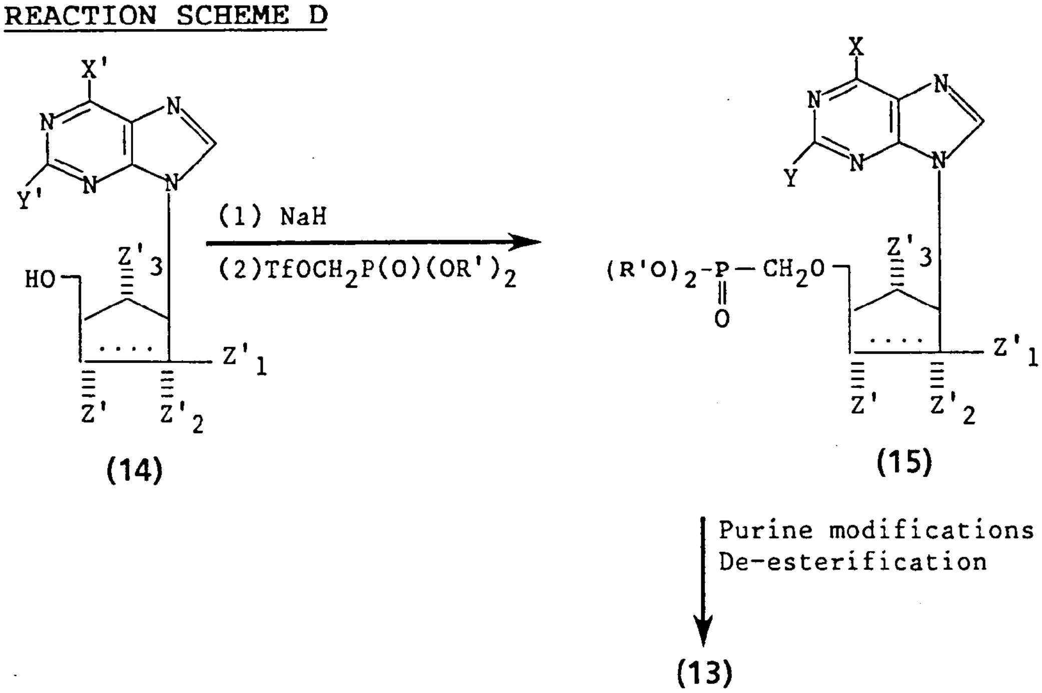


(14) (15) (13)

wherein X′, Y′, Z, $Z_1$, $Z_2$, $Z_3$, X, Y and R′ are as previously defined.

In effecting the foregoing reaction the intermediates (14) are first activated with NaH or KH to its alkoxide in anhydrous tetrahydrofuran at about 20°C under an inert atmosphere (argon) which, when reacted with a triflate [diethyl-(trifluoromethylsulfonylmethane)phosphonate] or the corresponding tosylate (i.e., $TosOCH_2P(O)(OEt)_2$) or with $TfOCH_2P(O)(OEt)_2$, in the presence of the catalyst 18-Crown-6 at -15° to 0°C in tetrahydrofuran or dimethoxyethane. Following these reactions the de-esterification and modifications to the pyrimidine and purine nuclei may be effected.

Of course, it is obvious from the nucleoside art that the compounds of Formula I may also be prepared by reacting the purine and pyrimidine bases with a cyclopentene derivative wherein the $R_1Q$ moiety is already attached thereto. However, such procedures are generally followed only in special situations, depending upon the specific definitions of the X, Y, Z and Q moieties, and of course of other factors such as the ready availability

of the appropriate starting materials, reactants, and the volume capacity of compound to be produced; all such factors being known and appreciated by those skilled in the art.

To prepare compounds of this invention having the subgeneric formula

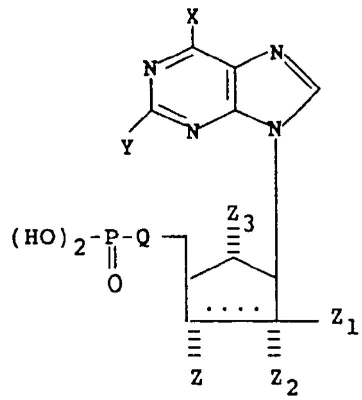

(16)

wherein X, Y and Q are as previously defined and one of Z or $Z_2$ is F or OH, and the other is H, or both $Z_1$ and $Z_2$ are F, the following reaction scheme may be utilized

REACTION SCHEME E

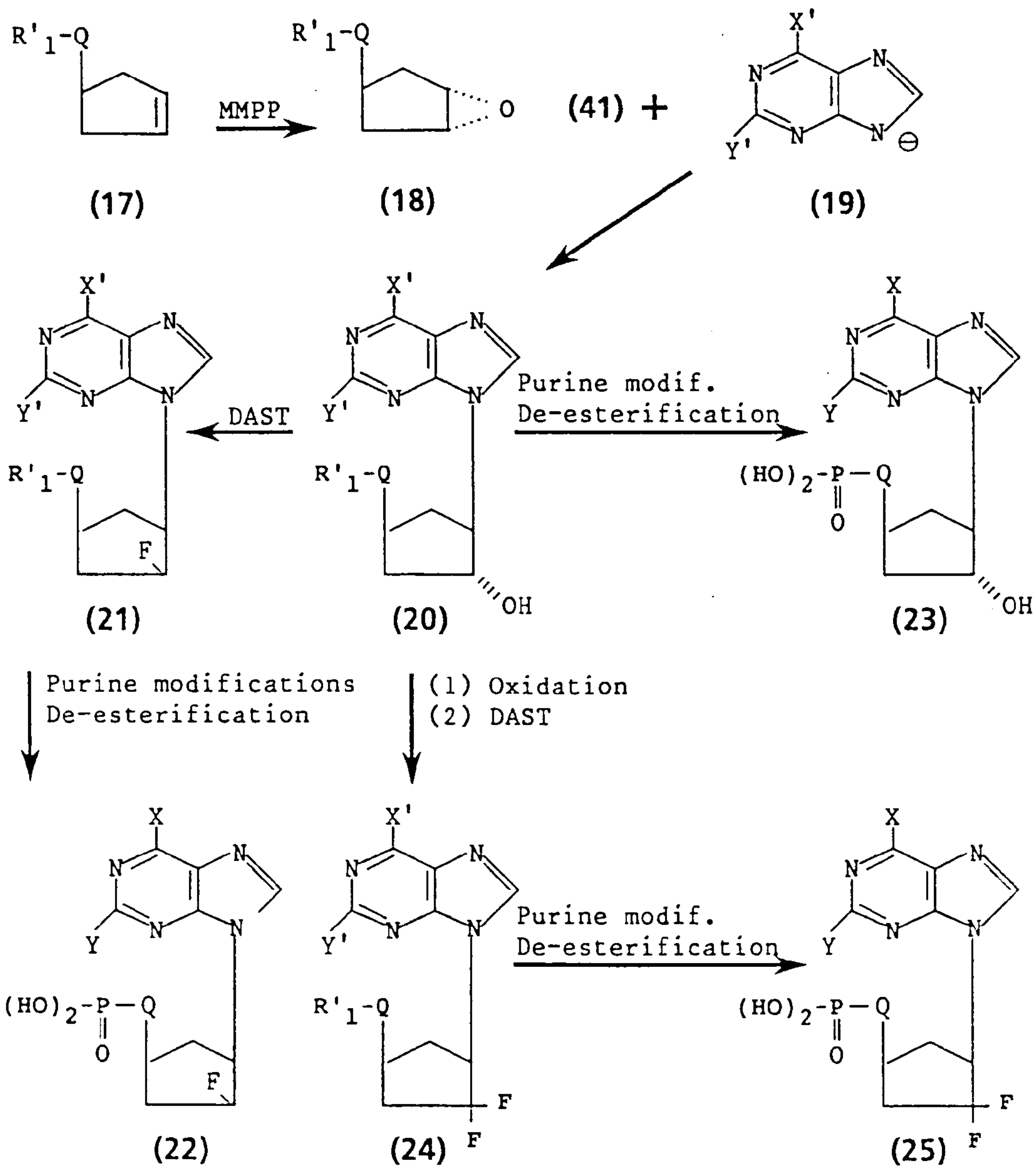


(17) (18) (19)

(21) (20) (23)

(22) (24) (25)

wherein $R'_1$ is $P(O)(OC_{1-6}$ alkyl) and X′, Y′, X, Y and Q are as previously defined.

In the foregoing reaction the formation of the epoxide (18) is preferably effected by using magnesium monoperoxyphthalate (MMPP) and the epoxide is condensed with an activated purine (19) at 100°C to 140°C in the presence of dimethylformamide to produce compounds (20) which, when treated with DAST, form the corresponding 3′-fluoro analog (21) which may then be subjected to any necessary modifications to the X′ and Y′ and de-esterified with TMSBr as previously detailed to produce compounds (22). Compounds (20) may also be subjected to the modifications of the purine moiety and to de-esterification to produce compounds (23). Alternatively, compounds (20) may be oxidized and the resulting *in situ* intermediates treated with DAST to form the 3′,3′-difluoro analogs (24) which are subjected to the purine modifications and de-esterification procedures to produce compounds (25).

To prepare compounds of this invention having the sub-generic formula

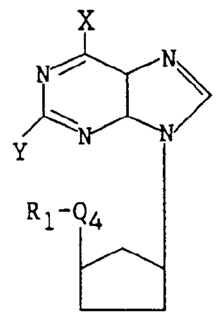

(26)

wherein X, Y and $R_1$ are as previously defined and $Q_4$ is

$CH_2OCH_2-$ or $-CH_2\overset{A}{\underset{A}{C}}$ with A being as previously defined (i.e., $R_1Q_4$ is $-CH_2OCH_2P(O)(OR')_2$ or $-CH_2\overset{A}{\underset{A}{C}}\ P(O)(OR')_2$), the processes of the following reaction scheme may be utilized.

REACTION SCHEME F

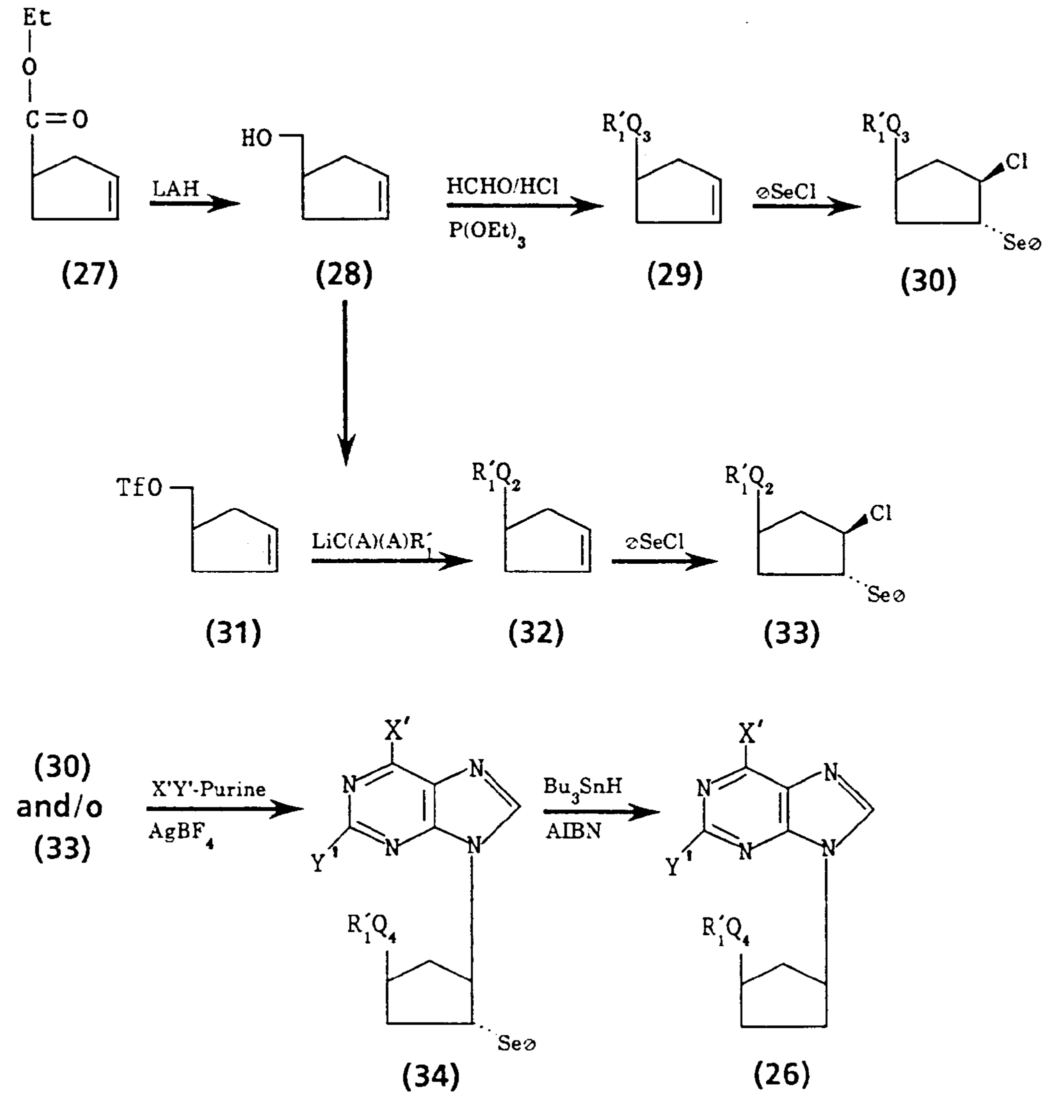


(27) (28) (29) (30)

(31) (32) (33)

(30) and/o (33)

(34) (26)

wherein X', Y', $R_1'Q_4$ are as previously defined, $R_1'Q_2$ is $(EtO)_2(O)P-C(A)(A)-CH_2$, $R_1'Q_3$ is $(EtO)_2(O)PCH_2OCH_2$ and ∅ is phenyl.

The process is initiated by reducing the ethyl ester of 4-carboxy cyclopentene (27) with lithium aluminum hydride (LAH) to the corresponding hydroxymethyl analog (28). Sequential treatment of (28) with formaldehyde (preferably paraformaldehyde) in the presence of HCl, followed by treatment of the resulting product with an ester of phosphite [preferably $P(OEt)_3$] will yield compounds of formula (II). Alternatively compounds (28) may be activated by reaction with triflic anhydride in the presence of pyridine and the resulting compounds (31) reacted with a lithio derivative of the diethyl ester of the appropriately A,A-di-substituted phosphonic acid, i.e., Li-$C(A)(A)P(O)(OEt)_2$, such as for example the lithio derivative of the diethyl ester of difluoromethyl phosphonic acid [i.e., Li-$CF_2P(O)(OEt)_2$] to produce compounds (32). Following the preparation of compounds (29) and (32), they are treated with phenylselenenyl chloride (∅SeC1) in anhydrous dichloromethane under argon at about 20°C to yield intermediates (30) and (33) which are condensed with a 6-X',2-Y'-purine in the presence of calcium carbonate and silver tetrafluoroborate ($AgBF_4$) in nitromethane at about 20°C under argon to produce compounds (34). The phenylselenyl moiety (Se∅) is removed by reaction with tributyltin hydride ($Bu_3SnH$) in the presence of azoisobutyronitrile (AIBN) to produce compounds (26) which may then be de-esterified and the

desired modifications to the purine and pyrimidine substituents effected as previously described to produce the compounds

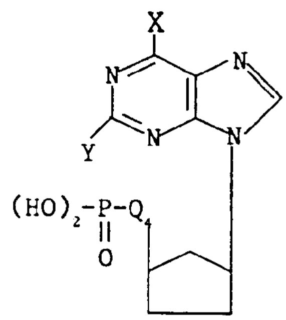

(35)

To prepare compounds of this invention having the subgeneric formula

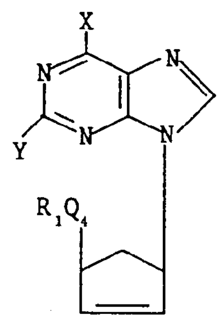

(36)

wherein X, Y, $Q_4$ and $R_1$ are as previously defined, the processes illustrated by the following reaction scheme may be utilized.

REACTION SCHEME G

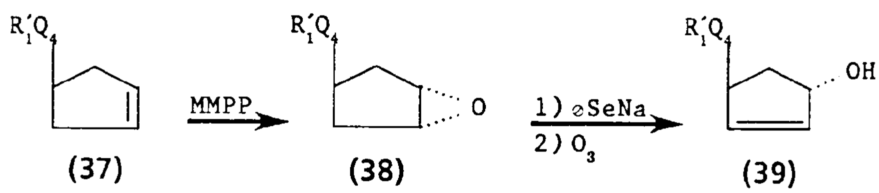


(37) (38) (39)

wherein $R_1'$ and $Q_4$ are as previously defined.

Compounds (37) may be converted to their respective allylic alcohols by formation of an epoxide oxidation with meta-chloroperbenzoic acid (MCPBA) or preferably, from a safety point of view, with magnesium monoperoxyphtalate (MMPP), followed by an opening of the epoxide (38) by reaction with sodium selenium phenylate followed by treatment with ozone at temperatures of about -78°C to +20°C in $CH_2Cl_2$ to produce the desired stereoisomeric form of compounds (39). With less certainty to obtain the desired stereoisomeric form of the desired allylic alcohol (39), compounds (37) may be subjected to a one-step oxidation using $^1O_2$ oxygen. Once obtained, compounds (39) may be condensed with the X′, Y′, W′, V′ purine and pyrimidine base compounds using either the Mitsunobu or the triflic anhydride series of reactions as previously described, followed by the above-described reactions to modify the X, Y, W and V substituents of the purine and pyrimidine bases. Compounds of formula (36) may also be conveniently prepared by transforming the alcohol of compounds (23) into a mesylate (by reaction with mesyl chloride in the presence of pyridine) or into a triflate (by reaction with triflic anhydride and pyridine in $CH_2Cl_2$) followed by an elimination reaction in the presence of a base (preferably DBU or potassium $^t$butoxide).

To prepare compounds of this invention having the subgeneric formula

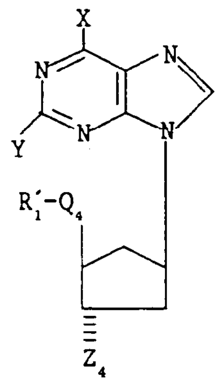

**(40)**

wherein X and Y are as previously defined and $Z_4$ is $N_3$ or F, the processes illustrated in the following reaction scheme may be utilized.

REACTION SCEME H

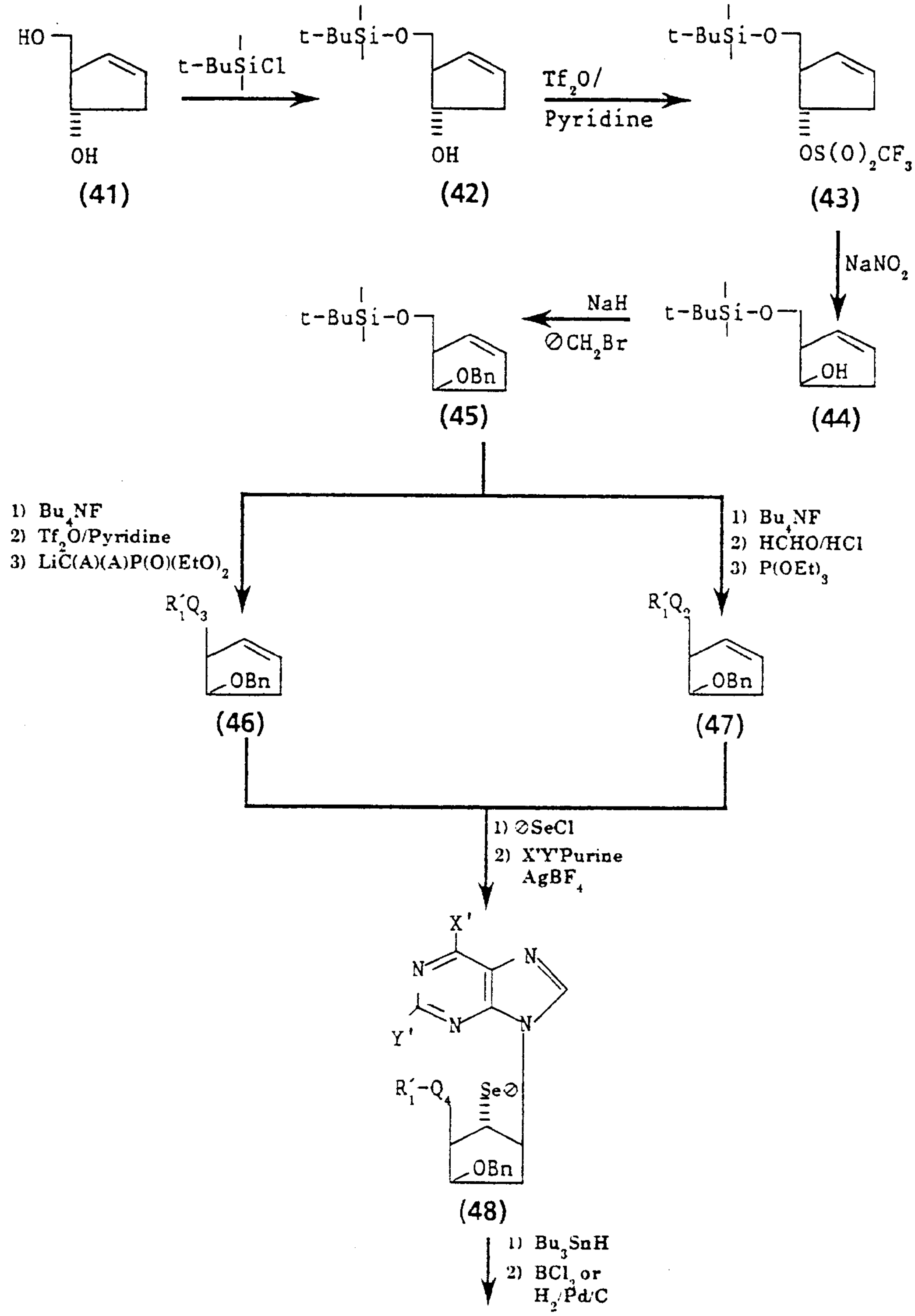

REACTION SCHEME H (cont'd)

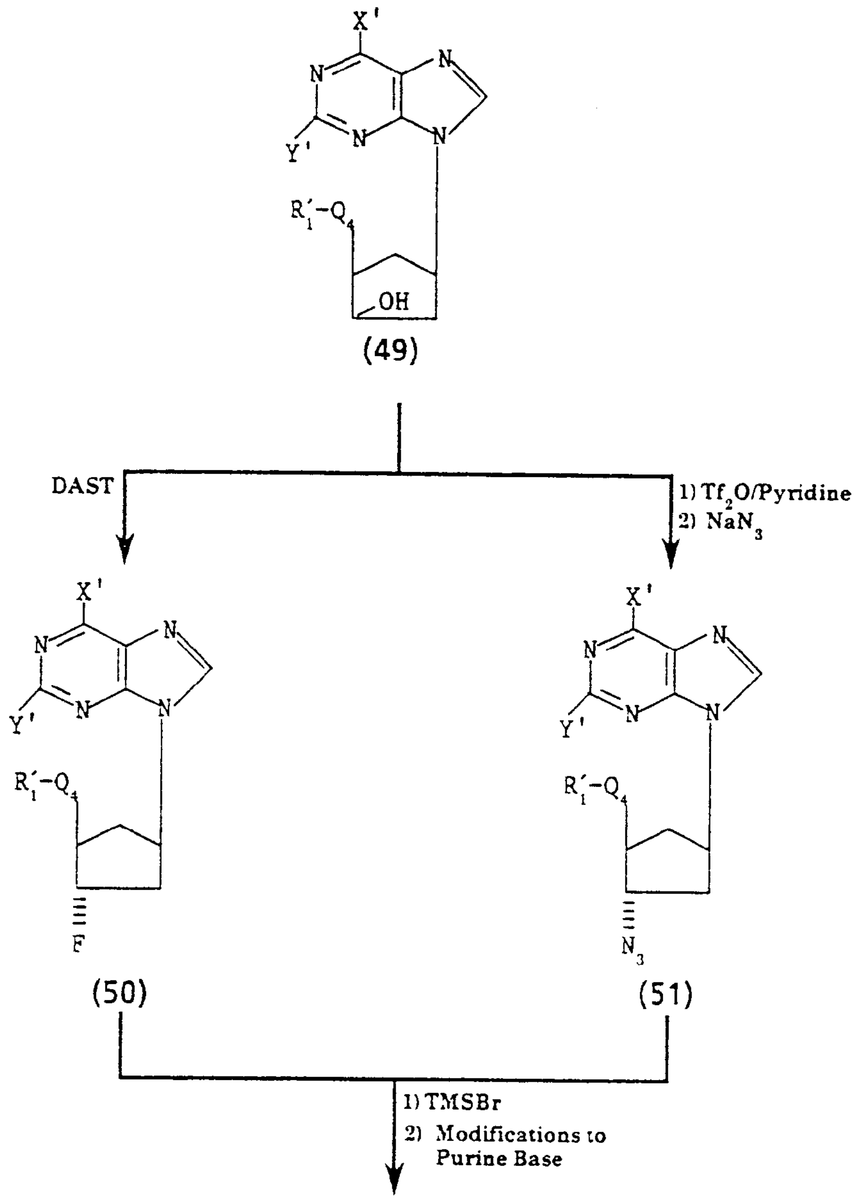


(49)

(50)

(51)

(40)

wherein X', Y', X, Y, $R_1'Q_4$, $R_1'Q_2$, and $R_1'Q_3$ are as previously defined, Bn represents benzyl and t-buSi represents t-butyl dimethylsilyl.

The reaction series is initiated by the selective protection of the primary alcohol of compound (41) by reaction with demethyl-t-butyl silylchloride to produce compound (42) which, by sequential treatment with triflic anhydride in the presence of pyridine and then treatment of compounds (43) with sodium nitrite ($NaNO_2$), inverts the alcohol which is then protected with a benzyl protecting group by reaction with benzyl bromide ($\varnothing CH_2Br$) to yield compounds (45). Following the preparation of compounds (45), the t-butyl-dimethylsilyl protecting group is removed by treatment with tetrabutylammonium fluoride in tetrahydrofuran and, as in Reaction Scheme F, the so-deprotected analog of compound (45) is alternatively (a) sequentially treated with $Tf_2O$/pyridine and then with $LiC(A)(A)P(O)(EtO)_2$ to produce compounds (46) or (b) sequentially treated with HCHO/HCl and then

$P(OEt)_3$ to produce compounds (47). Again, as analogously described in Reaction Scheme F, compounds (46) and (47) are sequentially reacted with phenylselenium chloride and the products thereof condensed with the X′,Y′-purine base to produce compounds (48). Treatment of compounds (48) with tributyltin hydride in the presence of AIBN, as previously described, removes the phenylselenyl (Se∅) moiety. Hydrolysis of the resulting product with either hydrogen in the presence of palladium on carbon or preferably with boron trichloride removes the benzyl protecting group to produce compounds (49). Again, as analogously described above, treatment of compounds (49) with $Tf_2O$ in the presence of pyridine and then with sodium azide produces the azides of compounds (51). Following preparation of compounds (50) and (51), compounds are de-esterified with TMSBr and followed by the desired modifications to the X,Y-substituents of the purine base and W,V-substituents of the pyrimidine base. Of course, in those instances wherein it is desired to modify the purine base bearing the alkyl esters, the modifications may be effected without the de-esterification with TMSBr.

The following examples illustrate the preparation of the compounds of this invention.

## EXAMPLE 1

### Preparation of [2-[3-(6-Amino-9H-purin-9-yl)cyclopentyl]-1,1-difluoroethyl]phosphonic acid

Step A:

Synthesis of 3-Cyclopentenylmethanol

0.78 mol (109.45 g) of 3-cyclopentenylcarboxylic acid ethyl ester dissolved in 400 ml of anhydrous ether is added dropwise to a stirred suspension of 24 g of lithium aluminum hydride (0.7 mmol) in 500 ml of anhydrous ether. The reaction mixture is stirred at 20°C for 3 hours and hydrolyzed by careful addition of 24 ml of water, 24 ml of 15% aqueous sodium hydroxide and three times 24 ml of water. The white precipitate is discarded by filtration and the filtrate is washed twice with brine, dried over sodium sulfate, filtered and evaporated to give 67.5 g of the expected product 3-cyclopentenylmethanol (88% yield) which is used in the next step without further purification.

Step B:

Synthesis of Trifluoromethylsulfonyloxy-3-cyclopentenyl-methane

20 ml of triflic anhydride (120 mmol) are added dropwise to a stirred solution of 11 ml of pyridine dissolved in 45 ml of anhydrous dichloromethane at -10°C under argon. The white suspension is stirred at -10°C for 30 minutes, cooled at -20°C and 6 g of 3-cyclopentenylmethanol dissolved in 20 ml of dichloromethane are slowly added to the reaction mixture which is stirred at 0°C for 4 hours, hydrolyzed with cold water. The organic phase is rapidly washed three times with cold saturated aqueous ammonium chloride and brine, dried over sodium sulfate, filtered and evaporated to give 13.07 g of a pale brown oil which is used in the next step without further purification.

Step C:

Synthesis of 2-[3-Cyclopenten-1-yl]1,1-difluoroethylphosphonic acid, diethyl ester

100 mmol (18.8 g) of difluoromethyl phosphonic acid, diethyl ester dissolved in 60 ml of tetrahydrofuran are slowly added to a stirred solution of lithium diisopropylamide (100 mmol) in 60 ml of anhydrous tetrahydrofuran, at -78°C under argon. The reaction mixture is stirred at -78°C for 35 minutes and 57 mmol (13.07 g) of trifluoromethylsulfonyloxy-3-cyclopentenylmethane dissolved in 60 ml of tetrahydrofuran are slowly added at -78°C. After 6 hours at -78°C and 2 hours at 20°C, the reaction mixture is quenched by addition of 50 ml of saturated aqueous ammonium chloride and evaporated under reduced pressure. The residue is dissolved in 300 ml of ethyl acetate, washed with water and brine, dried over sodium sulfate, filtered, evaporated and purified by flash chromatography on silica gel using petroleum ether and increasing amounts of ethyl acetate as eluents giving 7.9 g of the expected product 2-[3-cyclopenten-1-yl]-1,1-difluoroethylphosphonic acid, diethyl ester, (52% yield).

Step D:

Synthesis of [2-[3-(6-Chloro-9H-purin-9-yl)-4-phenylselenylcyclopentyl]-1,1-difluoroethyl]phosphonic acid, diethyl ester

3.56 g (18.7 mmol) of phenylselenenyl chloride are added portionwise to a stirred solution of 5 g (18.7 mmol) of 2-[3-cyclopentenyl]1,1-difluoroethylphosphonic acid, diethyl ester in 30 ml of anhydrous dichloromethane at 20°C under argon. After 6 hours at 20°C, the reaction mixture is evaporated under reduced pressure and the residue is dissolved in 30 ml of nitromethane and treated successively by 19 mmol (2.94 g) of 6-chloropurine, 1.8 g of calcium carbonate and 4 g of silver tetrafluoroborate at 20°C under argon. After 48 hours at 20°C, the orange-green suspension is evaporated under reduced pressure and directly purified by flash chromatography on silica gel using chloroform and increasing amounts of methanol as eluents giving 3.17 g of the expected product [2-[3-(6-chloro-9H-purin-9-yl)-4-phenylselenylcyclopentyl]-1,1-difluoroethyl]phosphonic acid, diethyl ester (30% yield).

Step E:

Synthesis of [2-[3-(6-Chloro-9H-purin-9-yl)cyclopentyl]-1,1-difluoroethyl]phosphonic acid, diethyl ester

2.64 ml of tributyltinhydride (9.8 mmol) are added to a stirred solution of [2-[3-(6-chloro-9H-purin-9-yl)-4-phenylselenylcyclopentyl]-1,1-difluoroethyl]phosphonic acid, diethyl ester (2.83 g, 4.9 mmol) and 160 mg of azoisobutyronitrile (AIBN) dissolved in 30 ml of toluene. The reaction mixture is stirred at 75-80°C for 5 hours and at 20°C for 20 hours, evaporated under reduced pressure and directly purified by flash chromatography on silica gel giving 1.26 g (3 mmol) of [2-[3-(6-chloro-9H-purin-9-yl)cyclopentyl]-1,1-difluoroethyl]phosphonic acid, diethyl ester.

Step F:

Synthesis of [2-[3-(6-Amino-9H-purin-9-yl)cyclopentyl]-1,1-difluoroethyl]phosphonic acid

1.6 ml (12 mmol) of trimethylsilylbromide is added to a stirred solution of 1.25 g of [2-[3-(6-chloro-9H-purin-9-yl)cyclopentyl]-1,1-difluoroethyl]phosphonic acid, diethyl ester dissolved in 20 ml of anhydrous dichloromethane at 20°C under argon. The pale brown solution is evaporated under reduced pressure, the residue is dissolved in anhydrous acetonitrile and an orange solid is precipitated upon addition of 0.25 ml of water. The solid is collected by filtration and suspended in 80 ml of methanol saturated with ammonia in a steel cylinder which is heated at 100°C for 20 hours. The reaction mixture is cooled at 20°C and a white solid is collected by filtration. The title compound [2-[3-(6-amino-9H-purin-9-yl)cyclopentyl]-1,1-difluoroethyl]phosphonic acid is obtained after recrystallization from methanol (430 mg).

**EXAMPLE 2**

**Preparation of {[3-(6-Amino-9H-purin-9-yl)cyclopentyl]methoxymethyl}phosphonic acid**

Step A:

Synthesis of 3-[(Cyclopenten-1-yl)methoxymethyl]phosphonic acid, diethyl ester

Hydrochloric acid gas is vigorously bubbled into a solution of 10 g of 3-cyclopentenylmethanol and 3 g of paraformaldehyde in 100 ml of anhydrous dichloromethane at 0°C for 10 minutes. Gentle hydrochloric acid bubbling is continued at 0°C for 4 hours; excess hydrochloric acid is removed by bubbling nitrogen and the resulting solution is evaporated under reduced pressure giving 13 g of an oil which is added to 15.3 ml of triethylphosphite. The resulting mixture is stirred at 70°C for 20 hours. The crude product (21 g) is then directly purified by flash chromatography on silica gel using petroleun ether and increasing amounts of ethyl acetate as eluents giving 14.56 g of the expected product 3-[(cyclopenten-1-yl)methoxymethyl]phosphonic acid, diethyl ester (61% yield).

Step B:

Synthesis of {[3-(6-chloro-9H-purin-9-yl)-4-phenylselenylcyclopentyl]methoxymethyl} phosphonic acid, diethyl ester

2.6 g of {[3-(6-chloro-9H-purin-9-yl)-4-phenylselenylcyclopentyl]methoxymethyl} phosphonic acid, diethyl ester have been prepared from 5 g of 3-[(cyclopenten-1-yl)methoxy methyl]phosphonic acid, diethyl ester, 3.9 g of phenylselenenyl chloride, 4.3 g of silver tetrafluoroborate, 4.6 g of 6-chloropurine and 2 g of calcium carbonate according to the method described in Example 1, Step D.

Step C:

Synthesis of {[3-(6-chloro-9H-purin-9-yl)-cyclopentyl]methoxymethyl} phosphonic acid, diethyl ester

1.4 g of {[3-(6-chloro-9H-purin-9-yl)-cyclopentyl]methoxymethyl} phosphonic acid, diethyl ester is prepared from 2.32 g of {[3-(6-chloro-9H-purin-9-yl)-4-phenylselenylcyclopentyl]methoxymethyl} phosphonic acid, diethyl ester, 2.32 ml of tributyltinhydride and 120 mg of AIBN according to the method described in Example 1, Step D.

Step D:

Synthesis of {[3-(6-Amino-9H-purin-9-yl)cyclopentyl]methoxymethyl}phosphonic acid.

600 mg of the final product {[3-(6-amino-9H-purin-9-yl)cyclopentyl]-methoxymethyl}phosphonic acid is obtained in two steps from {[3-(6-chloro-9H-purin-9-yl)-cyclopentyl]methoxymethyl} phosphonic acid, diethyl ester according to the method described in Example 1, Step F.

**EXAMPLE 3**

**Preparation of {2-[4-(2-Amino-1,6-dihydro-6-oxo-9H-purin-9-yl)-2-cyclopenten-1-yl]ethyl}phosphonic acid**

Step A:

Synthesis of [3-Cyclopenten-1-yl)ethyl]phosphonic acid, diethyl ester

100 mmol of n-butyllithium (60 ml of a 1.65N solution in hexane) are added to a stirred solution of 100 mmol (15.2 g) of methyl phosphonic acid, diethyl ester dissolved in 60 l of tetrahydrofuran at -78°C under argon. After stirring at -78°C for 1 hour, 75 mmol of [(trifluoromethylsulfonyloxy)cyclopenten-4-yl]methane (17.2 g) dissolved in 75 ml of tetrahydrofuran are added dropwise to the reaction mixture which is stirred at -78°C for 1 hour, at -40°C for 2 hours and quenched at 0°C by addition of 100 ml of saturated aqueous ammonium chloride. The crude mixture is evaporated under reduced pressure; the residue is suspended in 350 ml of ethyl acetate, washed with water and brine, dried over sodium sulfate, filtered, evaporated and purified by flash chromatography on silica gel giving 45 mmol (10.44 g) of [3-cyclopenten-1-yl)ethyl]phosphonic acid, diethyl ester.

Step B:

Synthesis of [2-Cyclopent[b]oxiran-4-yl)ethyl]phosphonic acid, diethyl ester

20 mmol (9.88 g) of magnesium monoperoxyphtalate dissolved in 40 ml of water are added dropwise to a stirred solution of [3-cyclopenten-1-yl)ethyl]phosphonic acid, diethyl ester (30 mmol, 6.96 g) in 30 ml of isopropanol at 20°C. The reaction mixture is stirred at 20°C for 5 hours and treated with 20 ml of an aqueous saturated thiosulfate solution. The crude mixture is evaporated and extracted with three portions of 100 ml ethyl acetate. The organic layers are gathered, washed twice with bicarbonate and brine, dried over sodium sulfate, filtered, evaporated and purified by flash chromatography on silica gel giving 20 mmol of the expected product [2-cyclopent[b]oxiran-4-yl)ethyl]phosphonic acid, diethyl ester (66% yield) as well as 5 mmol of the other isomer.

Step C:

Synthesis of [2-(4-Hydroxy-2-cyclopenten-1-yl)ethyl]phosphonic acid, diethyl ester

0.86 g of sodium borohydride is added to a stirred solution of diphenyldiselenide (2.8 g, 9 mmol) in 30 ml of ethanol. After stirring at 20°C for 1 hour, [2-cyclopent-[b]oxiran-4-yl)ethyl]phosphonic acid, diethyl ester (15 mmol, 3.72 g) is added to the reaction mixture which is stirred at 20°C for 20 hours, quenched with acetone (2 ml) and acetic acid (1 ml) and evaporated under reduced pressure to give a crude yellow oil which is dissolved in ethyl acetate (150 ml), washed with bicarbonate and brine, dried over sodium sulfate, filtered and evaporated to give 6.05 g of a crude oil. This oil is dissolved in 25 ml of anhydrous dichloromethane at -78°C and oxidized by bubbling ozone in the solution until a blue coloration appears. Nitrogen is then bubbled through the solution and 1.2 ml of triethylamine is added at -78°C. The reaction mixture is slowly heated to 20°C, stirred for 3 hours, evaporated under reduced pressure and directly purified by flash chromatography on silica gel using ethyl acetate as eluent to give 2.65 g (10 mmol) of [2-(4-hydroxy-2-cyclopenten-1-yl)ethyl]-phosphonic acid, diethyl ester (66% yield).

Step D:

Synthesis of [2-[4-(2-Amino-6-chloro-9H-purin-9-yl)-2-cyclopenten-1-yl]ethyl]phosphonic acid, diethyl ester

3.5 ml of triflic anhydride (20 mmol) are added dropwise to a stirred solution of 1.85 ml of pyridine in 8 ml of anhydrous dichloromethane at -10°C under argon. After 30 minutes, 2.65 g (10 mmol) of [2-(4-hydroxy-2-cyclopenten-1-yl)ethyl]phosphonic acid, diethyl ester dissolved in 10 ml of dichloromethane are added to the reaction mixture at -20°C under argon. After 3 hours at -20°C, the reaction mixture is diluted with cold dichloromethane and rapidly washed with iced-water, cold saturated ammonium chloride and brine, dried over sodium sulfate, filtered and evaporated to give 4.1 g of crude triflate which is dissolved in 5 ml of dimethylformamide and added to a stirred suspension of 10 mmol (1.69 g) of 2-amino-6-chloropurine and 11 mmol of potassium carbonate in 25 ml of dimethylformamide at 0°C under argon. The reaction mixture is stirred at 20°C for 20 hours, evaporated under reduced pressure and directly purified by flash chromatography on silica gel, affording 6 mmol (2.4 g) of [2-[4-(2-amino-6-chloro-9H-purin-9-yl)-2-cyclopenten-1-yl]ethyl]-phosphonic acid, diethyl ester (60% yield).

Step E:

Synthesis of {2-[4-(2-Amino-1,6-dihydro-6-oxo-9H-purin-9-yl)-2-cyclopenten-1-yl]ethyl}phosphonic acid

22 mmol (3. ml) of trimethylsilylbromide are added to a stirred solution of [2-[4-(2-amino-6-chloro-9H-purin-9-yl)-2-cyclopenten-1-yl]ethyl]phosphonic acid, diethyl ester (2.4 g, 6 mmol) in 25 ml of anhydrous dichloromethane at 20°C under argon. The reaction mixture is stirred at 20°C for 30 hours and evaporated to dryness. The residue is dissolved in acetonitrile and a pale orange solid is precipitated by addition of water. The solid is collected by filtration, dissolved in 20 ml IN HCl and 5 ml tetra-hydrofuran and heated at 90-95°C for 18 hours. A white precipitate is formed when the reaction mixture is cooled to 0°C and the final compound {2-[4-(2-amino-1,6-dihydro-6-oxo-9H-purin-9-yl)-2-cyclopenten-1-yl] ethyl} phosphonic acid is isolated after recrystallization from water (0.81 g, 2.15 mmol).

**EXAMPLE 4**

**Preparation of [2-[3-(6-Amino-9H-purin-9-yl)-4-hydroxycyclopentyl]ethyl] phosphonic acid**

Step A:

Synthesis of (Iodomethyl)-3-cyclopenten

200 mmol (27.8 ml) of triethylamine and 2 mmol of DMAP (0.24 g) are added to a solution of 19.6 g of 3-cyclopentenylmethanol (prepared as described in Example 1, Step A) in 200 ml of anhydrous dichloromethane at 20°C under argon. The reaction mixture is cooled at -10°C and a solution of p-toluenesulfonylchloride (38.1 g, 200 mmol) in 200 ml of anhydrous dichloromethane is added dropwise. A white precipitate is rapidly formed and the reaction mixture is stirred at 20°C for 20 hours, filtered, concentrated under reduced pressure and the

residue is purified by flash chromatography on silica gel (petroleum ether and ether in a ratio 9 to 1 as eluents) to give 39.63 g of pure intermediate which is dissolved in 200 ml of acetone containing 35 g of sodium iodide. The mixture is heated at refluxing temperature for 6 hours, cooled to 20°C, filtered and carefully evaporated. The residue is purified by rapid filtration on silica gel using petroleum ether as solvent and distilled (75°C/15 mmHg) to give 33 g of the expected product.

Step B:

Synthesis of 2-[3-Cyclopenten-1-yl]ethyl phosphonic acid, diisopropyl ester

65.6 ml (100 mmol) of n-butyllithium (1.54 M solution in hexane) are solwly added to a stirred solution of diisopropyl methane phosphonate (18 g, 100 mmol) in 100 ml of anhydrous tetrahydrofuran at -78°C under argon. The reaction mixture is stirred at 0°C for 20 minutes and (iodomethyl)-3-cyclopenten (20.8 g, 100 mmol) is added dropwise. The reaction mixture is stirred at 0°C for 20 hours, quenched with saturated aqueous ammonium chloride and evaporated under reduced pressure. The residue is extracted with ether, the organic layers are washed with brine, dried over sodium sulfate, concentrated and purified by flash chromatography on silica gel (eluent: ethyl acetate) to give 10.9 g of the expected product.

Step C:

Synthesis of [2-[3-(6-Amino-9H-purin-9-yl)-4-hydroxycyclopentyl]ethyl] phosphonic acid, diisopropyl ester

Magnesium monoperphtalate (8.72 g, 15 mmol) in 45 ml of water is added dropwise to a solution of 2-[3-cyclopenten-1-yl]ethyl phosphonic acid, diisopropyl ester (6.5 g, 25 mmol) in 30 ml of isopropanol at 20°C. The reaction mixture is stirred for 6 hours, quenched with aqueous saturated sodium thiosulfate (50 ml) and concentrated under reduced pressure. The residue is extracted with ethyl acetate (3 x 30 ml). The organic layers are washed with bicarbonate, dried over sodium sulfate, evaporated under reduced pressure and purified by flash chromatography on silica gel using chloroform/methanol (9/l) to give 5.45 g of the expected epoxide intermediate as a pale yellow oil. This oil is dissolved in 10 ml of dimethylformamide and added to a stirred solution of the sodium salt of adenine (prepared by reacting 1 equivalent of sodium hydride with 2.23 g of adenine in dimethylformamide at 20°C for 30 minutes) and the reaction mixture is stirred at 140°C for 24 hours. Dimethylformamide is then evaporated under reduced pressure and the residue is purified by flash chromatography on silica gel using chloroform/methanol (9/l) as eluent, to give 3.18 g of the expected product.

Step D:

Synthesis of [2-[3-(6-Amino-9H-purin-9-yl)-4-hydroxycyclopentyl]ethyl] phosphonic acid

[2-13-(6-amino-9H-purin-9-yl)-4-hydroxy-cyclopentyl]ethyl] phosphonic acid, diisopropyl ester (2.34 g, 5.7 mmol) is dissolved in 10 ml of acetonitrile and treated with 3 ml (23 mmol) of trimethylsilyl bromide at 20°C under argon. The reaction mixture is stirred at 20°C for 20 hours and concentrated under reduced pressure. The residue is redissolved in 10 ml of acetonitrile, quenched by addition of 0.45 ml of water and evaporated to give a white solid which is recrystallized from hot water to give 1.25 g of the title compound.

**EXAMPLE 5**

**Preparation of [2-[3-(6-Amino-9H-purin-9-yl)-4-fluorocyclopentyl]ethyl phosphonic acid**

Step A:

Synthesis of [2-[3-(6-Amino-9H-purin-9-yl)-4-fluorocyclopentyl]ethyl phosphonic acid, diisopropyl ester

Diethylaminosulfurtrifluoride (DAST, 6 mmol, 0.75 ml) is added dropwise to a stirred solution of [2-[3-(6-amino-9H-purin-9-yl)-4-hydroxy-cyclopentyl]ethyl] phosphonic acid, diisopropyl ester (4 mmol, 1.64 g) in 10 ml of anhydrous dichloromethane at 20°C under argon. The reaction mixture is stirred at 20°C for 20 hours, cooled to -15°C and carefully quenched with 3 ml of methanol. The crude mixture is evaporated under reduced pressure and the residue is directly purified by flash chromatography on silica gel using chloroform/methanol (95/5) as eluent, to give 1.05 g of the expected product.

Step B:

Synthesis of [2-[3-(6-Amino-9H-purin-9-yl)-4-fluorocyclopentyl]ethyl phosphonic acid

The title compound is obtained from the corresponding diisopropyl ester (described in Step A) by the method described in Example 4, Step D.

**EXAMPLE 6**

**Preparation of {[3-(6-Cyclopropylamino-9H-purin-9-yl)cyclopentyl]methoxymethyl} phosphonic acid**

12 mmol (1.6 ml) of trimethylsilylbromide are added to a stirred solution of 1.35 g of {[3-(6-chloro-9H-purin-9-yl)cyclopentyl]methoxymethyl} phosphonic acid, diethyl ester (prepared as described in Example 2, Step C) dissolved in 20 ml of anhydrous dichloromethane at 20°C under argon. The yellow solution is evaporated under reduced pressure, the residue is dissolved in anhydrous acetonitrile and a pale yellow solid is precipitated upon addition of 0.25 ml of water. The solid is collected by filtration and suspended in 80 ml of methanol containing 5 g of cyclopropylamine in a steel cylinder which is heated at 100°C for 20 hours. The reaction mixture is cooled at 20°C and a white solid is collected by filtration. The expected final compound {[3-(6-cyclopropylamino-9H-purin-9-yl)-cyclopentyl]methoxymethyl}phosphonic acid is obtained in a pure form after two recrystallizations from hot methanol (380 mg).

**EXAMPLE 7**

**Preparation of {[4-(2-Amino-1,6-dihydro-6-oxo-9H-purin-9-yl)-2-cyclopenten-1-yl]methoxymethyl} phosphonic acid**

Step A:

Synthesis of {[4-(2-amino-1,6-dihydro-6-oxo-9H-purin-9-yl)-2-cyclopenten-1-yl]methoxymethyl}phosphonic acid, diethyl ester

10 mmol of carbovir (2.5 g) are added portionwise to a stirred suspension of sodium hydride (30 mmol, 1.2 g, 60% in oil, washed three times with anhydrous hexane) in 35 ml of anhydrous dimethylsulfoxide at 20°C under argon. The reaction mixture is stirred at 20°C for 30 minutes and 20 mmol (5.30 g) of diethyl(trifluoromethylsulfonyloxymethane)phosphonate dissolved in 20 ml of dimethylsulfoxide are slowly added to the mixture which is stirred at 20°C for 20 hours, quenched with saturated aqueous ammonium chloride and evaporated to dryness under reduced pressure. The residue is suspended in 100 ml of 0.1N HCl solution in water. The aqueous phase is washed 5 times with 35 ml portions of ether, stirred on charcoal, filtered and evaporated. The residue is dissolved in hot water and 1.59 g of the expected product {[4-(2-amino-1,6-dihydro-6-oxo-9H-purin-9-yl)-2-cyclopenten-1-yl]methoxymethyl} phosphonic acid, diethyl ester is obtained by precipitation on cooling (40% yield).

Step B:

Synthesis of {[4-(2-amino-1,6-dihydro-6-oxo-9H-purin-9-yl)2-cyclopenten-1-yl]methoxymethyl}phosphonic acid

18 mmol (2.4 ml) of trimethylsilylbromide are added to a stirred solution of 1.59 g (4 mmol) of {[4-(2-amino-1,6-dihydro-6-oxo-9H-purin-9-yl)-2-cyclopenten-1-yl]methoxymethyl}phosphonic acid, diethyl ester in 25 ml of anhydrous dimethylformamide at 20°C under argon. The reaction mixture is stirred at 20°C for 36 hours and evaporated to dryness. The crude residue is dissolved in 30 ml of acetonitrile and the final compound is obtained as a white solid on addition of 0.5 ml of water. Further recrystallization from hot water gives 800 mg of the expect compound {[4-(2-amino-1,6-dihydro-6-oxo-9H-purin-9-yl)-2-cyclopenten-1-yl]methoxymethyl}phosphonic acid.

## EXAMPLE 8

**Preparation of [2-[4-(2-Amino-6-hydroxy-9H-purin-9-yl)-3-fluoro-2-hydroxycyclopentyl]ethenyl phosphonic acid**

Step A:

Synthesis of (±)(1α,2β,3α,4α)-4-(2-Amino-6-chloro-9H-purin-9-yl)-3-fluoro-2-hydroxycyclopentanemethanol-($^t$butyldimethyl)silyl ether

$^t$Butyldimethylsilylchloride (22 mmol, 3.3 g) is added to a solution of imidazole (45 mmol, 3.6 g) and (±)(1α,2β,3α,4α)-4-(2-amino-6-chloro-9H-purin-9-yl)-3-fluoro-2-hydroxycyclopentanemethanol (20 mmol, 6.02 g) in 60 ml of anhydrous dimethylformamide at 20°C. The reaction mixture is stirred for 2 hours, evaporated under reduced pressure and the residue is purified by flash chromatography on silica gel to give 5.85 g of the expected product (70% yield).

Step B:

Synthesis of (±)(1α,2β,3α,4α)-4-(2-Acetylamino-6-chloro-9H-purin-9-yl)-3-fluoro-2-O-acetyloxy-cyclopentane methanol

5.85 g of (±)(1α,2β,3α,4α)-4-(2-amino-6-chloro-9H-purin-9-yl)-3-fluoro-2-hydroxycyclopentanemethanol-($^t$butyldimethyl)silyl ether are dissolved in pyridine (50 ml) and treated with 50 mmol of acetic anhydride. The reaction mixture is stirred at 60°C for 18 hours and evaporated under reduced pressure. The crude residue is purified by flash chromatogranhy on silica gel (using ethylacetate as eluent) to give 6.8 g of the expected intermediate which is dissolved in 50 ml of tetrahydrofuran and stirred with 40 mmol of tetrabutylammonium fluoride. After 25 hours the reaction mixture is evaporated to dryness and the residue is purified by flash chromatography on silica gel to give 4.76 g of the expected product.

Step C:

Synthesis of [2-[4-(2-Acetylamino-6-chloro-9H-purin-9-yl)-3-fluoro-2-O-acetyloxy-cyclopentyl]ethenyl phosphonic acid diethyl ester

4.76 g of(±)(1α,2β,3α,4α)-4-(2-acetylamino-6-chloro-9H-purin-9-yl)-3-fluoro-2-O-acetyloxy-cyclopentane methanol, dissolved in 30 ml of anhydrous dichloromethane, are added dropwise to a suspension of pyridinium chlorochromate (3.01 g, 15 mmol) in 15 ml of dichloromethane at 20°C under argon. The reaction mixture is vigorously stirred for 4 hours, filtered over celite, evaporated and purified rapidly on florisil (dichloromethane is used as eluent) to give 3.78 g of a crude product (after evaporation) which is dissolved in 15 ml of anhydrous tetrahydrofuran and added, at -15°C, to a stirred solution of the sodium salt of tetraethylmethylene diphosphonate (prepared at 0°C by reacting 20 mmol of NaH with 20 mmol of tetraethylmethylene diphosphonate in 25 ml tetrahydrofuran for 40 minutes). The reaction mixture is stirred at 20°C for 20 hours, quenched with saturated aqueous ammonium chloride, evaporated under reduced pressure and purified by flash chromatography on silica gel (eluent: chloroform/methanol: 85/15) to give 3.6 g of the expected product as a white solid.

Step D:

Synthesis of [2-[4-(2-Amino-6-hydroxy-9H-purin-9-yl)-3-fluoro-2-hydroxycyclopentyl]ethenyl phosphonic acid

3.6 g of [2-[4-(2-acetylamino-6-chloro-9H-purin-9-yl)-3-fluoro-2-O-acetyloxy-cyclopentyl]ethenyl phosphonic acid diethyl ester dissolved in 20 ml of anhydrous acetonitrile are treated with 23 mmol (3 ml) of trimethylsilylbromide at 20°C under argon. The reaction mixture is stirred at 20°C for 20 hours and evaporated under reduced pressure. The sticky residue is dissolved in acetonitrile and reacted with methanol and then evaporated. The obtained white solid is suspended in methanol and reacted with 10 mg of sodium methoxide at 20°C overnight. The reaction mixture is evaporated and the residue is dissolved in 1H HCl and heated at 100°C for 20 hours. The title compound (1.6 g) is obtained after evaporation and 2 subsequent recrystallizations from water.

The compounds of this invention are useful in the medical therapy particularly for the treatment or

prophylaxis of viral infections such as for example hepatitis B, retroviral infections, especially AIDS, respiratory syncitial viruses, and herpes viruses such as herpes simplex I and II, cytomegalovirus and varicella-zoster-virus.

Examples of retroviral infections which may be treated or prevented in accordance with the invention include human retroviral infections such as Human Immunodeficiency Virus (HIV), HIV-2 and Human T-cell Lymphotropic Virus (HLTV) e.g. HTLV-I or HTLV-IV infections. The compounds according to the invention are especially useful for the treatment or prophylaxis of AIDS and related clinical conditions such as AIDS-related complex (ARC), progressive generalized lymphadenopathy (PGL), AIDS-related neurological conditions, such as multiple sclerosis or tropical paraparesis, anti-HIV antibody-positive and HIV-positive conditions, Kaposi's sarcoma and thrombocytopenic purpura. The compounds may also be used in the treatment or prevention of psoriasis.

In a further aspect of the present invention there is included: a) a method for the treatment and prophylaxis of retroviral infections and hepatitis B infections which comprises treating the subject with a therapeutically effective amount of a compound according to the invention; b) use of a compound according to the invention in the manufacture of a medicament for the treatment or prophylaxis of any of the above-mentioned infections or conditions.

The compounds of this invention may be employed in combination with other therapeutic agents for the treatment or prophylaxis of the above infections or conditions. Examples of such further therapeutic agents include agents that are effective for the treatment or prophylaxis of viral infections or associated conditions such as 3′-azido-3′-deoxythymidine (zidovudine), 2′,3′-dideoxynucleosides such as 2′,3′-dideoxycytidine, 2′,3′-dideoxy adenosine and 2′,3′-dideoxyinosine, acyclic nucleosides (e.g. acyclovir), interferons such as $\alpha$-interferon, renal excretion inhibitors such as probenicid, nucleoside transport inhibitors such as dipyridamole, as well as immunomodulators such as interleukin II and granulocyte macrophage colony stimulating factors. The component compounds of such combination therapy may be administered simultaneously, in either separate or combined formulations, or at different times, e.g. sequentially such that a combined effect is achieved.

The compounds according to the invention, also referred to herein as the active ingredient, may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the chosen active ingredient.

In general a suitable dose will be in the range of 3.0 to 120 mg per kilogram body weight of the recipient per day, preferably in the range of 6 to 90 mg per kilogram body weight per day and most preferably in the range of 15 to 60 mg per kilogram body weight per day. The desired dose is preferably presented as two, three, four, five, six or more sub-doses administered at appropriate intervals throughout the day. These sub-doses may be administered in unit dosage forms, for example, containing 10 to 1,500 mg, preferably 20 to 1,000 mg, and most preferably 50 to 700 mg of active ingredient per unit dosage form.

Ideally, the active ingredient should be administered to achieve peak plasma concentrations of the active compound of from about 1 to about 75$\mu$M, preferably about 2 to 50$\mu$M, most preferably about 3 to about 30$\mu$M. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible for the active ingredient to be administered alone it is preferable to present it as a pharmaceutical formulation. The formulations of the present invention comprise at least one active ingredient, as defined above, together with one or more acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredients with the carrier which constitutes one or more accessory ingredients. In general, the formulations are prepared by uniformly and intimately bringing into association the active ingredient with liquid-carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste. A tablet may be made by compression or moulding, optionally with one or more accessory ingredients. Compressed tablets may be prepared by compressing in a suitable machine the active ingredient in a free-flowing form such as a powder or granules, optionally mixed with a binder (p.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach. This is particularly advantageous for purine nucleoside derivatives as such compounds are susceptible to acid hydrolysis.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain anti-oxydants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above-recited, or an appropriate fraction thereof, of an active ingredient.

As is true for many large classes of compounds suitable for use as therapeutic agents certain subclasses and certain specific members of the generic class will be found to be more effective than others. In this instance, the preferred B moiety is purine. The preferred $R_1$-Q moieties are $CH_2OCH_2P(O)(OH)_2$, $CH_2CH_2P(O)(OH)2$ and $CH=CHP(O)(OH)_2$. The preferred A moieties are H (most preferred) and F. The preferred Z moiety is H, F or $N_3$. The preferred $Z_1$-$Z_2$ moieties are H, F or OH. The preferred $Z_3$ moiety is H, F or OH. The preferred X moieties are OH, Cl or $NH_2$ and the preferred Y moieties are H or $NH_2$. The preferred W moieties are $NH_2$ or OH and the preferred V moieties are H, $CH_3$, I, Cl or -CH=CHBr. Of course, for the phosphonate R group of Formula I, H is preferred.

Preferred specific compounds are those of the following chart wherein $R_1$ is $P(O)(OH)_2$.

| Q | Z | $Z_1,Z_2$ | $Z_3$ | X | Y | W | V |
|---|---|---|---|---|---|---|---|
| $CH_2OCH_2$ | H | H,H | H | $NH_2$ | H | - | - |
| $CH_2OCH_2$ | $N_3$ | H,H | H | OH | $NH_2$ | - | - |
| $CH_2OCH_2$ | $N_3$ | H,H | H | - | - | OH | $CH_3$ |
| $CH_2OCH_2$ | F | H,H | H | - | - | OH | Cl |
| $CH_2OCH_2$ | H | H,OH | H | OH | $NH_2$ | - | - |
| $CH_2CH_2$ | H | H | H | $NH_2$ | H | - | - |
| $CH_2CH_2$ | H | H,OH | H | $NH_2$ | H | - | - |
| $CH_2CH_2$ | H | H,F | H | $NH_2$ | H | - | - |
| $CH_2CH_2$ | $N_3$ | H | H | - | - | OH | $CH_3$ |
| $CH_2CH_2$ | $N_3$ | H | F | - | - | OH | $CH_3$ |
| $CH_2CH_2$ | H | H* | H | - | - | OH | $CH_3$ |
| $CH_2CH_2$ | H | H,H | H | - | - | $NH_2$ | H |
| $CH_2CH_2$ | H | H* | H | OH | $NH_2$ | - | - |
| CH=CH | H | H* | H | OH | $NH_2$ | - | - |
| CH=CH | F | H,H | H | $NH_2$ | H | - | - |
| CH=CH | $N_3$ | H,H | H | OH | $NH_2$ | - | - |
| CH=CH | H | H,OH | H | $NH_2$ | H | - | - |
| CH=CH | H | H,F | H | OH | $NH_2$ | - | - |
| CH=CH | $N_3$ | H | H | - | - | OH | $CH_3$ |
| CH=CH | H | H* | H | - | - | $NH_2$ | H |
| CH=CH | H | H,H | F | - | - | OH | H |
| CH=CH | H | H,H | H | $NH_2$ | H | - | - |
| CH=CH | H | H | OH | OH | $NH_2$ | - | - |

* = unsaturated

## Claims

**1.** A compound of the formula

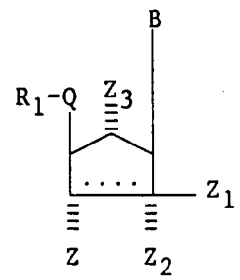

I

the tautomers, optical and geometric isomers and mixtures thereof, and the pharmaceutically acceptable salts thereof, wherein

B is a purine or a pyrimidine moiety of the subformulae

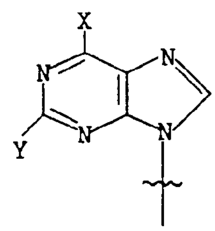

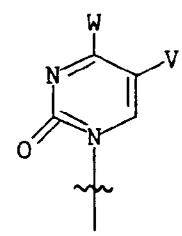

the depicted wavy line illustrating the point of attachment of the B moiety to the remaining portion of Formula I, and the dotted line represents a facultative double bond,

X is $-OR_2$, $-N(R_2)(R_2)$, Cl, -SH or $-SCH_3$, with $R_2$ being H, $C_{1-6}$ alkyl, or $C_{3-6}$ cycloalkyl,

Y is H or $NH_2$,

W is $NH_2$, OH or $-OC_{1-3}$ alkyl,

V is H, $C_{1-3}$ alkyl, halogeno, $-N(R_2)(R_2)$, -SH, $-SCH_3$, -C≡CH, $-CH=CH_2$, or CH=CHBr,

Z is H, F or $N_3$, with the proviso that when the dotted line represents a double bond then Z is H, and each of $Z_1$, $Z_2$ and $Z_3$ is H, F, or OH, with the proviso that when one of $Z_1$ or $Z_2$ is OH the other is H, and with the further proviso that when the dotted line is a double bond, $Z_1$ is H or F and $Z_2$ is deleted,

$R_1$-Q is $-CH_2OCH_2P(O)(OR)_2$, $-CH_2C(A)(A)P(O)(OR)_2$, or $-CH=C(A)P(O)(OR)_2$, and A is H, F or Cl, and R is H or $C_{1-6}$ alkyl.

**2.** A compound of Claim 1 wherein B is a purine moiety.

**3.** A compound of Claim 1 wherein B is a pyrimidine moiety.

**4.** A compound of Claim 2 wherein X is OH, Cl or $NH_2$, and Y is H or $NH_2$.

**5.** A compound of Claim 3 wherein W is $NH_2$ or OH, and V is H, $CH_3$, I, Cl or -CH=CHBr.

**6.** A compound of Claim 1 wherein B is a purine moiety, $R_1$-Q is $-CH_2OCH_2P(O)(OH)_2$, X is $NH_2$ or OH, Y is $NH_2$ or H, Z is $N_3$ or H, $Z_1$ is H, $Z_2$ is OH or H, and $Z_3$ is H.

**7.** A compound of Claim 1 wherein B is a pyrimidine moiety, $R_1$-Q is $-CH_2OCH_2P(O)(OH)_2$, V is $CH_3$ or Cl, W is OH, Z is $N_3$, H or F, and each of $Z_1$, $Z_2$ and $Z_3$ is H.

**8.** A compound of Claim 1 wherein B is a purine moiety, $R_1$-Q is $CH_2CH_2P(O)(OH)_2$, X is $NH_2$ or OH, Y is $NH_2$ or H, Z is H or $N_3$, and each of $Z_1$, $Z_2$ and $Z_3$ is H.

**9.** A compound of Claim 1 wherein B is a pyrimidine moiety wherein $R_1$-Q is $-CH_2OCH_2P(O)(OH)_2$, V is $CH_3$ or H, W is OH or $NH_2$, Z is $N_3$ or H, and each of $Z_1$, $Z_2$ and $Z_3$ is H.

**10.** A compound of Claim 1 wherein B is a purine moiety wherein $R_1$-Q is -CH=CHP(O)(OH)$_2$, X is $NH_2$ or OH, Y is $NH_2$ or H, Z is $N_3$ or H, $Z_1$ and $Z_2$ are H, and $Z_3$ is H or OH.

**11.** A compound of Claim 1 wherein B is a pyrimidine moiety wherein $R_1$-Q is -CH=CHP(O)(OH)$_2$, V is $CH_3$ or H, W is OH or $NH_2$, Z is $N_3$ or H, $Z_1$ and $Z_2$ are H, and $Z_3$ is F or H.

**12.** A compound of Claim 1, said compound being selected from the group consisting of
[2-[3-(6-amino-9H-purin-9-yl)cyclopentyl]-1,1-difluoroethyl]phosphonic acid;
{[3-(6-amino-9H-purin-9-yl)cyclopentyl]-methoxymethyl}phosphonic acid;
{2-[4-(2-amino-1,6-dihydro-6-oxo-9H-purin-9-yl)-2-cyclopenten-1-yl]ethyl}phosphonic acid;
[2-[3-(6-amino-9H-purin-9-yl)-4-hydroxy-cyclopentyl]ethyl] phosphonic acid;
[2-[3-(6-amino-9H-purin-9-yl)-4-fluorocyclopentyl]ethyl phosphonic acid;
{[3-(6-cyclopropylamino-9H-purin-9-yl)cyclopentyl]methoxymethyl} phosphonic acid;
{[4-(2-amino-1,6-dihydro-6-oxo-9H-purin-9-yl)-2-cyclopenten-1-yl]methoxymethyl} phosphonic acid;
[2-[4-(2-amino-6-hydroxy-9H-purin-9-yl)-3-fluoro-2-hydroxycyclopentyl]ethenyl phosphonic acid.

**13.** A process for preparing a compound of the formula

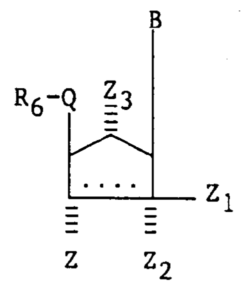

I

the tautomers, optical and geometric isomers and mixtures thereof, and the pharmaceutically acceptable salts thereof, wherein

B is a purine or a pyrimidine moiety of the subformulae

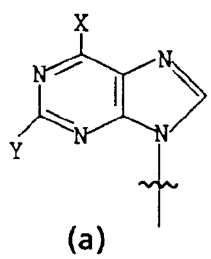

(a)

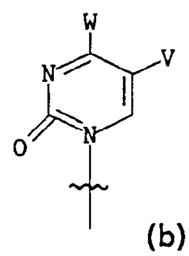

(b)

the depicted wavy line illustrating the point of attachment of the B moiety to the remaining portion of Formula I, and the dotted line represents a facultative double bond,

X is -O$R_2$, -N($R_2$)($R_2$), Cl, -SH or -S$CH_3$, with $R_2$ being H, $C_{1-6}$ alkyl, or $C_{3-6}$ cycloalkyl, and R is H or $C_{1-6}$ alkyl,

Y is H or $NH_2$,

W is $NH_2$, OH or -O$C_{1-3}$ alkyl,

V is H, $C_{1-3}$ alkyl, halogeno, -N($R_2$)($R_2$), -SH, -S$CH_3$, -C≡CH, -CH=$CH_2$, or CH=CHBr,

Z is H, F or $N_3$, with the proviso that when the dotted line represents a double bond then Z is H, and each of $Z_1$ , $Z_2$ and $Z_3$ is H, F, or OH, with the proviso that when one of $Z_1$ or $Z_2$ is OH the other is H, and with the further proviso that when the dotted line is a double bond, $Z_1$ is H or F and $Z_2$ is deleted,

$R_6$-Q is -$CH_2OCH_2$P(O)(OH)$_2$, -$CH_2$C(A)(A)P(O)(OH)$_2$, or -CH=C(A)P(O)(OH)$_2$, and A is H, F or Cl,

which comprises de-esterifying a compound of the formula

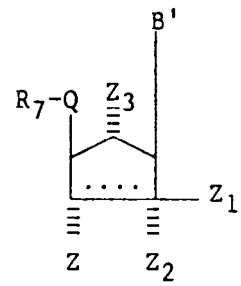

II

the tautomers, optical and geometric isomers and mixtures thereof, wherein Z, $Z_1$, $Z_2$ and $Z_3$ are as defined in Formula I,

$R_7$-Q is $-CH_2OCH_2P(O)(OC_{1-6}$ alkyl$)_2$, $-CH_2C(A)(A)P(O)(OC_{1-6}$ alkyl$)_2$, or $-CH=C(A)P(O)(OC_{1-6}$ alkyl$)_2$, and

B′ is a purine or a pyrimidine moiety of the formulae

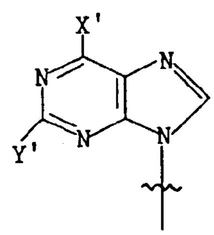

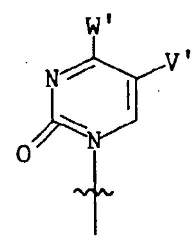

wherein

X′ is $-OR_2$, $-N(R_2)(R_2)$, Cl, -SH, $-SCH_3$, or a moiety transformable thereto,

Y′ is H, $NH_2$, or a moiety transformable thereto,

W is $NH_2$, OH, $-OC_{1-3}$ alkyl, or a moiety transformable thereto,

V′ is H, $C_{1-3}$ alkyl, halogeno, $-N(R_2)(R_2)$, -SH, $-SCH_3$, -C≡CH, -CH=CH2, CH=CHBr, or a moiety transformable thereto, and A and R are as defined for Formula I,

by reaction with TMSBr, and when necessary, chemically transforming a transformable moiety of X′, Y′, W′ or V′ to the desired X, Y, W or V moiety, respectively, followed by optionally forming the desired pharmaceutically acceptable salt thereof.

**14.** A Pharmaceutical composition comprising a compound of Claim 1, optionally in combination with a pharmaceutically acceptable carrier or excipient.

**15.** The derivatives according to claim 1 for use as pharmaceutically active compounds.

**16.** Use of the derivatives according to claim 1, for the preparation of drugs useful for the treatment or prophylaxis of viral infections.

European Patent Office

# EUROPEAN SEARCH REPORT

Application Number

EP 91 40 2022

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 369 409 (BRISTOL-MYERS SQUIBB CO.)<br>* The whole document *<br>----- | 1,14-16 | C 07 F 9/6561<br>A 61 K 31/675<br>C 07 F 9/6512 |
| | | | TECHNICAL FIELDS SEARCHED (Int. Cl.5) |
| | | | C 07 F 9/00<br>A 61 K 31/00 |

The present search report has been drawn up for all claims

| Place of search | Date of completion of the search | Examiner |
|---|---|---|
| THE HAGUE | 07-10-1991 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)